(19) Europäisches Patentamt
European Patent Office
Office européen des brevets

(11) Veröffentlichungsnummer : **0 092 174**
**B1**

(12) # EUROPÄISCHE PATENTSCHRIFT

(45) Veröffentlichungstag der Patentschrift : **05.03.86**

(51) Int. Cl.[4] : **C 07 J 1/00**, A 61 K 31/565// C07J21/00, C12P33/12

(21) Anmeldenummer : **83103605.8**

(22) Anmeldetag : **14.04.83**

(54) **11Beta-Chlor-Delta15-Steroide, Verfahren zu ihrer Herstellung und diese enthaltende pharmazeutische Präparate.**

(30) Priorität : **16.04.82 DE 3214690**

(43) Veröffentlichungstag der Anmeldung : **26.10.83 Patentblatt 83/43**

(45) Bekanntmachung des Hinweises auf die Patenterteilung : **05.03.86 Patentblatt 86/10**

(84) Benannte Vertragsstaaten : **AT BE CH DE FR GB IT LI LU NL SE**

(56) Entgegenhaltungen :
**FR-A- 2 326 927**
**NL-A- 7 209 299**
**US-A- 3 665 021**
**Die Akte enthält technische Angaben, die nach dem Eingang der Anmeldung eingereicht wurden und die nicht in dieser Patentschrift enthalten sind.**

(73) Patentinhaber : **SCHERING AKTIENGESELLSCHAFT Berlin und Bergkamen**
**Müllerstrasse 170/178 Postfach 65 03 11**
**D-1000 Berlin 65 (DE)**

(72) Erfinder : **Hofmeister, Helmut, Dr.**
**Weislingenstrasse 4**
**D-1000 Berlin 28 (DE)**
Erfinder : **Petzoldt, Karl, Dr.**
**Flachsweg 10**
**D-1000 Berlin 38 (DE)**
Erfinder : **Annen, Klaus, Dr.**
**Seegefelderstrasse 194**
**D-1000 Berlin 20 (DE)**
Erfinder : **Laurent, Henry, Dr.**
**Glambecker Weg 21**
**D-1000 Berlin 28 (DE)**
Erfinder : **Wiechert, Rudolf, Prof. Dr.**
**Petzowerstrasse 8 A**
**D-1000 Berlin 39 (DE)**
Erfinder : **Nishino, Yukishige, Dr.**
**Lanzendorfer Weg 14**
**D-1000 Berlin 22 (DE)**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

EP 0 092 174 B1

**Beschreibung**

Die Erfindung betrifft 11β-Chlor-$\Delta^{15}$-Steroide, Verfahren zu ihrer Herstellung und diese enthaltende pharmazeutische Präparate.

Die neuen 11β-Chlor-$\Delta^{15}$-Steroide werden durch die allgemeine Formel I gekennzeichnet

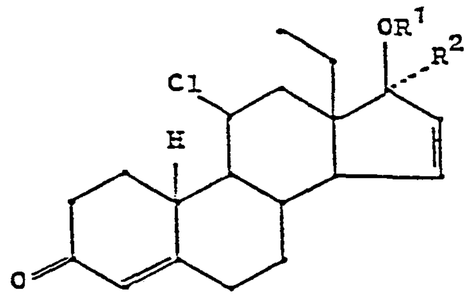

(I)

worin

$R^1$ ein Wasserstoffatom oder eine Acylgruppe einer organischen Carbonsäure mit bis zu 15 Kohlenstoffatomen und

$R^2$ eine Ethinyl-, Chlorethinyl- oder Propinylgruppe

bedeuten.

Die Acylgruppen $R^1$ gemäß Formel I leiten sich von Säuren ab, die in der Steroidchemie üblicherweise für Veresterungen angewendet werden. Bevorzugte Säuren sind organische Carbonsäuren mit bis zu 15 Kohlenstoffatomen, insbesondere niedere und mittlere aliphatische Carbonsäuren mit bis zu 7 Kohlenstoffatomen. Die Säuren können auch ungesättigt, verzweigt, mehrbasisch oder in üblicher Weise, zum Beispiel durch Hydroxy-, Acyloxy-, Alkoxy-, Oxo- oder Aminogruppen oder Halogenatome, substituiert sein. Geeignet sind auch cycloaliphatische, aromatische, gemischt aromatisch-aliphatische und heterocyclische Säuren, die ebenfalls in üblicher Weise substituiert sein können.

Beispielsweise seien folgende Carbonsäuren genannt: Ameinsensäure, Essigsäure, Propionsäure, Buttersäure, Isobuttersäure, Valeriansäure, Isovaleriansäure, Capronsäure, Önanthsäure, Caprylsäure, Pelargonsäure, Caprinsäure, Undecylsäure, Laurinsäure, Tridecylsäure, Myristinsäure, Pentadecylsäure, Trimethylessigsäure, Diethylessigsäure, tert.-Butylessigsäure, β-Cyclopentylpropionsäure, Cyclohexylessigsäure, Cyclohexancarbonsäure, Phenylessigsäure, Phenoxyessigsäure, Mono-, Di- und Trichloressigsäure, Aminoessigsäure, Diethylaminoessigsäure, Piperidinoessigsäure, Morpholinoessigsäure, Milchsäure, O-Tridecanoylglykolsäure, Bernsteinsäure, Adipinsäure, Benzoesäure, Nikotinsäure, Isonikotinsäure, Furan-2-carbonsäure.

Die neuen 11β-Chlor-$\Delta^{15}$-Steroide der allgemeinen Formel I werden gemäß Anspruch 11 durch Einführung des Restes $R^2$ in das 17-Oxo-Steroid der allgemeinen Formel II hergestellt.

Die Einführung des Restes $R^2$ kann nach bekannten Methoden mit einer metallorganischen Ethinyl-, Chlorethinyl- oder Propinylverbindung vorgenommen werden. Solche metallorganischen Verbindungen sind zum Beispiel Alkalimetallacetylide, wie zum Beispiel Kalium- und Lithiumacetylid, -chloracetylid bzw. -methylacetylid.

Die metallorganische Verbindung kann auch in situ gebildet und mit dem 17-Keton der Formel II zur Reaktion gebracht werden. So kann man zum Beispiel auf das 17-Keton in einem geeigneten Lösungsmittel Acetylen und ein Alkalimetall, insbesondere Kalium, Natrium oder Lithium, in Gegenwart eines $C_4$- oder $C_5$-Alkohols oder von Ammoniak oder in Form von zum Beispiel Butyllithium einwirken lassen. Lithiumchloracetylid kann aus 1,2-Dichlorethylen und einer etherischen Alkalimetallalkyl-, insbesondere Lithiummethyllösung gebildet werden.

Als metallorganische Ethinylverbindungen sind auch Ethinylmagnesium- oder Ethinylzinkhalogenide, insbesondere Ethinylmagnesiumbromid oder -jodid, geeignet.

Als Lösungsmittel werden insbesondere Dialkylether, Tetrahydrofuran, Dioxan, Benzol, Toluol usw. verwendet. Die Umsetzung erfolgt bei Temperaturen zwischen − 78 °C und + 50 °C.

Die sich gegebenenfalls anschließende Veresterung der 17-Hydroxygruppe erfolgt nach Methoden, die man üblicherweise in der Steroidchemie zur Veresterung tertiärer Hydroxygruppen anwendet. Als geeignete Veresterungsmethode sei beispielsweise die Umsetzung der Steroide mit Säureanhydriden oder Säurechloriden in Gegenwart basischer Katalysatoren, wie Natriumhydrogenkarbonat, Kaliumhydrogenkarbonat, Kaliumkarbonat, Natriumhydroxid, Kaliumhydroxid, Pyridin, Lutidin, Collidin, Triethylamin oder 4-Dimethylaminopyridin, genannt. Nach einer bevorzugten Ausführungsform wird die Veresterung in Gegenwart von Pyridin und 4-Dimethylaminopyridin durchgeführt.

Die Hydrolyse der 3-Oxoschutzgruppe, die vor oder auch nach der möglichen Veresterung erfolgen kann, wird nach den dem Fachmann bekannten Methoden durchgeführt. Für die Hydrolyse kommen Mineralsäuren, wie zum Beispiel Perchlorsäure, Schwefelsäure oder Salzsäure, oder organische Säuren, wie zum Beispiel Oxalsäure, in Betracht. Die Hydrolyse wird vorzugsweise in alkoholischer Lösung oder

in anderen polaren Lösungsmitteln, wie zum Beispiel Aceton, bei Temperaturen zwischen etwa 20 und 100 °C durchgeführt.

Die Oxoschutzgruppe X in Formel II bildet mit der (den) Doppelbindung(en), welche im Ring A oder B vorhanden sind, eine solche Anordnung von Atomen, daß die Verbindung durch Hydrolyse in ein 4,5-ungesättigtes 3-Oxosteroid umgewandelt wird. Nach einer bevorzugten Ausführungsform ist die 3-Oxogruppe durch Ketalbildung geschützt. Die Ketalreste leiten sich von den üblicherweise zum Schutz freier Oxogruppen verwendeten Alkoholen und Thioalkoholen ab, beispielsweise genannt seien: Ethylenglykol, 2,2-Dimethyl-propandiol-(1,3) und Ethandithiol-(1,2). Die 3-Oxogruppe kann aber auch durch Enoläther-, Enolester- oder Enaminbildung partiell geschützt werden.

Es ist bereits bekannt, daß 11β-Chlor-Steroide wertvolle biologische Eigenschaften besitzen. In der niederländischen Offenlegungsschrift 7209299 werden zum Beispiel 11β-Chlor-17α-ethinyl-18-methyl-4-östrene beschrieben, die eine starke gestagene Wirkung zeigen.

In der französischen Patentschrift 2 326 927 werden die zur vorliegenden Erfindung entsprechenden Verbindungen ohne den 11β-Chlor-Substituenten beschrieben.

Es wurde nun gefunden, daß die neuen 11β-Chlor-$\Delta^{15}$-Steroide der allgemeinen Formel I mindestens 3 mal stärker gestagen wirksam sind als die aus der NL 7209299 bekannten 11β-Chlor-Steroide.

Die gestagene Wirkung wurde im üblichen Clauberg-Test nach oraler Applikation an infantilen weiblichen Kaninchen ermittelt.

Die zur Erzielung eines positiven Effekts benötigte Mindestmenge wird durch den McPhail-Wert 1,5 ausgedrückt.

In der folgenden Tabelle werden die Ergebnisse des Tests zusammengestellt.

Tabelle

| | Verbindung | Dosis (mg) | McPhail |
|---|---|---|---|
| A | 11ß-Chlor-17α-ethinyl-17ß- | 0,1 | 3,1 |
| | hydroxy-18-methyl-4,15- | 0,03 | 2,9 |
| | östradien-3-on | 0,01 | 2,3 |
| | | 0,003 | 1,4 |
| B | 11ß-Chlor-17α-ethinyl-17ß- | 0,03 | 2,5 |
| | hydroxy-18-methyl-4-östren- | 0,01 | 1,4 |
| | 3-on | 0,003 | 1,1 |

Aus der Tabelle geht hervor, daß die Schwellendosis (McPhail 1,5) für die gestagene Wirkung bei der erfindungsgemäßen Verbindung A etwa bei 0,003 mg und bei den bekannten Verbindung B etwa bei 0,01 mg liegt.

Aufgrund der gestagenen Wirkung können die Verbindungen der allgemeinen Formel I zum Beispiel in Antikonzeptionspräparaten Verwendung finden, wobei sie als Gestagenkomponente in Kombination mit einer östrogen wirksamen Hormonkomponente, wie zum Beispiel Ethinylöstradiol, oder als alleinige Wirkkomponente eingesetzt werden. Die Verbindungen können aber auch in Präparaten zur Behandlung gynäkologischer Störungen verwendet werden.

Die neuen Verbindungen können mit den in der galenischen Pharmazie üblichen Zusätzen, Trägersubstanzen und Geschmackskorrigentien nach an sich bekannten Methoden zu den üblichen Arzneimittelformen verarbeitet werden. Für die orale Applikation kommen insbesondere Tabletten, Dragées, Kapseln, Pillen, Suspensionen oder Lösungen infrage. Für die parenterale Applikation kommen insbesondere ölige Lösungen, wie zum Beispiel Sesamöl- oder Rizinusöllösungen infrage, die gegebenenfalls zusätzlich noch ein Verdünnungsmittel, wie zum Beispiel Benzylbenzoat oder Benzylalkohol, enthalten können. Die Konzentration des Wirkstoffes ist abhängig von der Applikationsform. So enthalten beispielsweise Tabletten zur oralen Applikation vorzugsweise 0,01-0,5 mg Wirkstoff und Lösungen zur parenteralen Applikation vorzugsweise 1-100 mg Wirkstoff pro 1 ml Lösung.

Die Dosierung der erfindungsgemäßen Arzneimittel kann sich mit der Form und dem Zweck der Verabfolgung ändern. Beispielsweise liegt die tägliche kontrazeptive Dosis bei oraler Applikation bei 0,01-0,5 mg des neuen Gestagens, gegebenenfalls in Kombination mit 0,01-0,05 mg Ethinylöstradiol.

Die nach dem erfindungsgemäßen Verfahren eingesetzten Ausgangsverbindungen der allgemeinen Formel II können aus 11β-Chlor-18-methyl-4-östren-3,17-dion (niederländische Offenlegungsschrift 7209299) wie folgt hergestellt werden:

11β-Chlor-15α-hydroxy-18-methyl-4-östren-3,17-dion

Ein 2 Liter-Erlenmeyerkolben, der 500 ml einer 30 Minuten bei 120 °C im Autoklaven sterilisierten Nährlösung aus 3,0 % Glucose, 1,0 % Corn steep liquor, 0,2 % $NaNO_3$, 0,1 % $KH_2PO$ , 0,2 % $K_2HPO_4$, 0,05 % $MgSO_4 \cdot 7 H_2O$, 0,002 % $FeSO_4 \cdot 7 H_2O$ und 0,05 % KCl enthält, wird mit einer Schrägröhrchenkultur des Stammes Penicillium raistrickii (ATCC 10 490) beimpft und 2 1/2 Tage bei 30 °C auf einem Rotationsschüttler geschüttelt.

Mit 250 ml dieser Anzuchtkultur wird ein 20 Liter-Vorfermenter beimpft, der mit 15 l eines 60 Minuten bei 121 °C und 1,1 atü sterilisierten Mediums der gleichen Zusammensetzung wie die Anzuchtkultur gefüllt ist. Unter Zugabe von Silicon SH als Antischaummittel wird bei 29 °C und 0,7 atü Druck unter Belüftung (15 l/Min.) und Rühren (220 U/Min.) 24 Stunden germiniert.

Danach werden 0,9 l dieser Vorfermenterkultur unter sterilen Bedingungen entnommen und damit ein 20 Liter-Hauptfermenter beimpft, der mit 14 l eines wie oben sterilisierten Nährmediums der gleichen Zusammensetzung wie die Vorfermenterkultur beschickt ist. Nach einer Anwachsphase von 12 Stunden unter Vorfermenterbedingungen wird eine Lösung von 2,6 g 11β-Chlor-18-methyl-4-östren-3,17-dion in 130 ml Dimethylformamid hinzugegeben und weiter gerührt und belüftet. Der Verlauf der Fermentation wird durch Entnahme von Proben kontrolliert, die mittels Methylisobutylketon extrahiert und dünnschichtchromatographisch analysiert werden. Nach 14 Stunden Kontaktzeit ist die Umsetzung des Substrates beendet. Die Kulturbrühe wird filtriert, das Filtrat 3 mal mit je 10 l Methylisobutylketon extrahiert und die Extrakte mit dem Extrakt des gleichfalls mittels Methylisobutylketon extrahierten Mycels vereinigt. Nach Konzentrierung der Lösung im Umlaufverdampfer wird anschließend bei 50 °C Badtemperatur im Vakuum am Rotationsverdampfer zur Trockne eingeengt. Der ölig-kristalline Rückstand wird zur Aufreinigung über eine Kieselgelsäule chromatographiert und mittels des Lösungsmittelgradienten 5 l Methylenchlorid/3,5 l Methylenchlorid + 1,5 l Aceton eluiert. Die Hauptfraktion (2,28 g) wird schließlich aus Methylenchlorid/Isopropylether umkristallisiert, wobei man 1,8 g reines 11β-Chlor-15α-hydroxy-18-methyl-4-östren-3,17-dion vom Schmelzpunkt 169-170 °C erhält.

15α-Acetoxy-11β-chlor-18-methyl-4-östren-3,17-dion

1,6 g 11β-Chlor-15α-hydroxy-18-methyl-4-östren-3,17-dion in 5 ml Pyridin werden bei Raumtemperatur unter Argon mit 1,2 ml Acetanhydrid gerührt. Nach 18 Stunden wird die Lösung in salzäurehaltiges Eis/Wasser gegeben. Das ausgefallene Produkt wird abgesaugt, in Essigester gelöst und mit Wasser gewaschen. Nach Umkristallisieren des Rohproduktes aus Isopropylether erhält man 1,0 g 15α-Acetoxy-11β-chlor-18-methyl-4-östren-3,17-dion vom Schmelzpunkt 125,0 °C.

15α-Acetoxy-11β-chlor-3,3-(2′,2′-dimethyl-trimethylendioxy)-18-methyl-5-östren-17-on

1,3 g 15α-Acetoxy-11β-chlor-18-methyl-4-östren-3,17-dion in 13 ml Methylenchlorid und 1,3 ml o-Ameisensäuretriethylester werden bei Raumtemperatur mit 2,5 g 2,2-Dimethyl-1,3-propandiol und 15 mg p-Toluolsulfonsäure versetzt. Nach 7 Stunden verdünnt man mit Methylenchlorid, wäscht mit $NaHCO_3$-Lösung und Wasser und trocknet. Nach Chromatographieren des Rohproduktes an Kieselgel mit Hexan/Aceton (0-20 %) werden 800 mg 15α-Acetoxy-11β-chlor-3,3-(2′,2′-dimethyl-trimethylendioxy)-18-methyl-5-östren-17-on vom Schmelzpunkt 191,5 °C erhalten.

11β-Chlor-3,3-(2′,2′-dimethyl-trimethylendioxy)-18-methyl-5,15-östradien-17-on

600 mg 15α-Acetoxy-11β-chlor-3,3-(2′,2′-dimethyl-trimethylendioxy)-18-methyl-5-östren-17-on werden in 15 ml Dioxan mit 1 ml 1,5-Diazabicyclo [5,4,0] undec-5-en bei Raumtemperatur gerührt. Nach 1,5 Stunden wird die Lösung in Eis/Wasser gegeben. Das ausgefallene Produkt wird abfiltriert, in Essigester gelöst und mit Wasser gewaschen. Nach Chromatographieren des Rohproduktes an Kieselgel mit Hexan/Aceton (0-20 %) werden 300 mg 11β-Chlor-3,3-(2′,2′-dimethyl-trimethylendioxy)-18-methyl-5,15-östradien-17-on vom Schmelzpunkt 144,4 °C erhalten.

11β-Chlor-18-methyl-4,15-östradien-3,17-dion

300 mg 11β-Chlor-3,3-(2′,2′-dimethyl-trimethylendioxy)-18-methyl-5,15-östradien-17-on werden in 10 ml Aceton mit 0,2 ml halbkonzentrierter Salzsäure bei Raumtemperatur gerührt. Nach 45 Minuten neutralisiert man mit Natriumhydrogencarbonatlösung, engt das Gemisch im Vakuum ein, löst den Rückstand in Essigester, wäscht mit Wasser und trocknet über Natriumsulfat. Nach Umkristallisieren des Rohproduktes aus Aceton/Hexan erhält man 120 mg 11β-Chlor-18-methyl-4,15-östradien-3,17-dion.

Beispiel 1

a) 11β-Chlor-17α-ethinyl-3,3-(2′,2′-dimethyl-trimethylendioxy)-18-methyl-5,15-östradien-17β-ol

Acetylen wird 30 Minuten durch eine mit Eis/Wasser gekühlte Lösung von 90 ml Butyllithium (15 %ig

in Hexan) in 200 ml absolutem Tetrahydrofuran geleitet. Man gibt 2,6 g 11β-Chlor-3,3-(2′,2′-dimethyl-trimethylendioxy)-18-methyl-5,15-östradien-17-on in 30 ml Tetrahydrofuran zu und rührt unter Argon. Nach 30 Minuten versetzt man das Gemisch mit gesättigter Ammoniumchloridlösung, verdünnt mit Essigester und wäscht mit Wasser. Nach Chromatographieren des Rohproduktes an Kieselgel mit Hexan/Essigester (0-50 %) erhält man 1,4 g 11β-Chlor-17α-ethinyl-3,3-(2′,2′-dimethyl-trimethylendioxy)-18-methyl-5,15-östradien-17β-ol als Öl.

b) 11β-Chlor-17α-ethinyl-17β-hydroxy-18-methyl-4,15-östradien-3-on

1,4 g 11β-Chlor-17α-ethinyl-3,3-(2′,2′-dimethyl-trimethylendioxy)-18-methyl-5,15-östradien-17β-ol werden in 20 ml Aceton mit 0,5 ml halbkonzentrierter Salzsäure bei Raumtemperatur gerührt. Nach 45 Minuten neutralisiert man mit Natriumhydrogencarbonatlösung, engt das Gemisch im Vakuum ein, löst den Rückstand in Essigester, wäscht mit Wasser und trocknet über Natriumsulfat. Nach Umkristallisieren des Rohproduktes aus Aceton/Hexan erhält man 700 mg 11β-Chlor-17α-ethinyl-17β-hydroxy-18-methyl-4,15-östradien-3-on vom Schmelzpunkt 202,0 °C.

Beispiel 2

a) 11β-Chlor-17α-chlorethinyl-3,3-(2′,2′-dimethyltrimethylendioxy)-18-methyl-5,15-östradien-17β-ol

Zu 1,6 ml 1,2-Dichlorethylen in 12 ml abs. Ether werden bei 0 °C unter Argon 12 ml einer 5 %igen etherischen Methyllithiumlösung getropft. Nach 20 Minuten gibt man 650 mg 11β-Chlor-3,3-(2′,2′-dimethyl-trimethylendioxy)-18-methyl-5,15-östradien-17-on in einem Gemisch aus 15 ml Ether und 5 ml abs. Tetrahydrofuran zu und rührt bei Raumtemperatur. Nach 15 Minuten versetzt man vorsichtig mit gesättigter Ammoniumchloridlösung, verdünnt mit Ether, wäscht mit Wasser und trocknet. Nach Chromatographieren des Rohproduktes an Kieselgel mit Hexan/Aceton (0-20 %) werden 520 mg 11β-Chlor-17α-chlorethinyl-3,3-(2′,2′-dimethyl-trimethylendioxy)-18-methyl-5,15-östradien-17β-ol als schaumiges Produkt erhalten.

b) 11β-Chlor-17α-chlorethinyl-17β-hydroxy-18-methyl-4,15-östradien-3-on

400 mg 11β-Chlor-17α-chlorethinyl-3,3-(2′,2′-dimethyltrimethylendioxy)-18-methyl-5,15-östradien-17β-ol in 15 ml Aceton werden mit 0,5 ml halbkonzentrierter Salzsäure 1,5 Stunden bei Raumtemperatur gerührt. Das Gemisch wird mit Natriumhydrogencarbonatlösung neutralisiert und im Vakuum weitgehend eingeengt. Der Rückstand wird in Essigester gelöst, mit Wasser gewaschen und getrocknet. Nach Chromatographieren des Rohproduktes an Kieselgel mit Hexan/Aceton (0-20 %) werden 230 mg 11β-Chlor-17α-chlorethinyl-17β-hydroxy-18-methyl-4,15-östradien-3-on als schaumiges Produkt erhalten.

Beispiel 3

a) 11β-Chlor-3,3-(2′,2′-dimethyl-trimethylendioxy)-18-methyl-17α-(1-propinyl)-5,15-östradien-17β-ol

Methylacetylen wird 30 Minuten durch eine mit Eis/Wasser gekühlte Lösung von 20 ml Butyllithium (15 %ig in Hexan) in 60 ml abs. Tetrahydrofuran geleitet. Man gibt 1,1 g 11β-Chlor-3,3-(2′,2′-dimethyl-trimethylendioxy)-18-methyl-5,15-östradien-17-on in 10 ml Tetrahydrofuran zu und rührt unter Argon bei Raumtemperatur. Nach 1 Stunde tropft man gesättigte Ammoniumchloridlösung zu, verdünnt mit Essigester, wäscht mit Wasser und trocknet. Das Rohprodukt wird mit Hexan/Aceton (0-15 %) chromatographiert. Es werden 850 mg 11β-Chlor-3,3-(2′,2′-dimethyl-trimethylendioxy)-18-methyl-17α-(1-propinyl)-5,15-östradien-17β-ol als Ol erhalten.

b) 11β-Chlor-17β-hydroxy-18-methyl-17α-(1-propinyl)-4,15-östradien-3-on

Zu 760 mg 11β-Chlor-3,3-(2′,2′-dimethyl-trimethylendioxy)-18-methyl-17α-(1-propinyl)-5,15-östradien-17β-ol in 5 ml Aceton gibt man bei Raumtemperatur 0,1 ml halbkonzentrierte Salzsäure. Nach 30 Minuten neutralisiert man mit Natriumhydrogencarbonatlösung und engt das Gemisch im Vakuum weitgehend ein. Den Rückstand löst man in Essigester, wäscht mit Wasser und trocknet. Nach Chromatographieren des Rohproduktes mit Hexan/Aceton (0-15 %) erhält man 480 mg 11β-Chlor-17β-hydroxy-18-methyl-17α-(1-propinyl)-4,15-östradien-3-on als schaumiges Produkt.

Beispiel 4

17β-Acetoxy-11β-chlor-17α-ethinyl-18-methyl-4,15-östradien-3-on

700 mg 11β-Chlor-17α-ethinyl-17β-hydroxy-18-methyl-4,15-östradien-3-on in 10 ml Pyridin werden bei Raumtemperatur unter Zugabe von 100 mg 4-Dimethylaminopyridin mit 6 ml Acetanhydrid umgesetzt.

Das Gemisch wird nach 4 Stunden in essigsäurehaltiges Eis/Wasser gegeben. Das ausgefallene Produkt wird abgesauft, in Essigester gelöst und mit Wasser gewaschen. Das Rohprodukt wird an Kieselgel mit Hexan/Aceton (0-10 %) chromatographiert. Man erhält 510 mg 17β-Acetoxy-11β-chlor-17α-ethinyl-18-methyl-4,15-östradien-3-on als Schaum.

Beispiel 5

17β-Butyryloxy-11β-chlor-17α-ethinyl-18-methyl-4,15-östradien-3-on

300 mg 11β-Chlor-17α-ethinyl-17β-hydroxy-18-methyl-4,15-östradien-3-on in 5 ml Pyridin werden bei Raumtemperatur mit 2,0 ml Buttersäureanhydrid und 100 mg 4-Dimethylaminopyridin gerührt. Nach 6 Stunden gibt man das Gemisch in Eis/Wasser, extrahiert mit Methylenchlorid und wäscht mit Wasser. Nach Chromatographieren des Rohproduktes an Kieselgel mit Hexan/Aceton (0-10 %) erhält man 180 mg 11β-Chlor-17β-butyryloxy-17α-ethinyl-18-methyl-4,15-östradien-3-on als Öl.

Beispiel 6

11β-Chlor-17α-ethinyl-17β-heptanoyloxy-18-methyl-4,15-östradien-3-on

350 mg 11β-Chlor-17α-ethinyl-17β-hydroxy-18-methyl-4,15-östradien-3-on in 4 ml Pyridin versetzt man bei Raumtemperatur mit 2 ml Önanthsäureanhydrid und 60 mg 4-Dimethylaminopyridin. Nach 20 Stunden gibt man die Lösung in Eis/Wasser, extrahiert mit Methylenchlorid und wäscht mit Wasser. Nach Chromatographieren des Rohproduktes an Kieselgel mit Hexan/Aceton (0-8 %) werden 230 mg 11β-Chlor-17α-ethinyl-17β-heptanoyloxy-18-methyl-4,15-östradien-3-on als Öl erhalten.

Beispiel 7

11β-Chlor-17α-chlorethinyl-18-methyl-17β-propionyloxy-4,15-östradien-3-on

200 mg 11β-Chlor-17α-chlorethinyl-17β-hydroxy-18-methyl-4,15-östradien-3-on in 4 ml Pyridin werden bei Raumtemperatur mit 1,5 ml Propionsäureanhydrid und 20 mg 4-Dimethylaminopyridin gerührt. Nach 4 Stunden wird die Lösung in Eis/Wasser gegeben. Das ausgefallene Produkt wird abgesaugt, in Methylenchlorid gelöst, mit Wasser gewaschen und getrocknet. Nach Chromatographieren des Rohproduktes an Kieselgel mit Hexan/Aceton (0-8 %) erhält man 150 mg 11β-Chlor-17α-chlorethinyl-18-methyl-17β-propionyloxy-4,15-östradien-3-on als Öl.

Beispiel 8

17β-Acetoxy-11β-chlor-18-methyl-17α-(1-propinyl)-4,15-östradien-3-on

280 mg 11β-Chlor-17β-hydroxy-18-methyl-17α-(1-propinyl)-4,15-östradien-3-on in 4 ml Pyridin werden mit 2 ml Acetanhydrid und 30 mg 4-Dimethylaminopyridin bei Raumtemperatur gerührt. Nach 3 Stunden gibt man die Lösung in Eis/Wasser. Das ausgefallene Produkt wird abgesaugt, in Essigester gelöst, mit Wasser gewaschen und getrocknet. Nach Chromatographieren des Rohproduktes an Kieselgel mit Hexan/Aceton (0-10 %) erhält man 175 mg 17β-Acetoxy-11β-chlor-18-methyl-17α-(1-propinyl)-4,15-östradien-3-on als Schaum.

**Patentansprüche** (für die Vertragsstaaten : BE, CH, DE, FR, GB, IT, LI, LU, NL, SE)

1. 11β-Chlor-$\Delta^{15}$-Steroide der allgemeinen Formel I

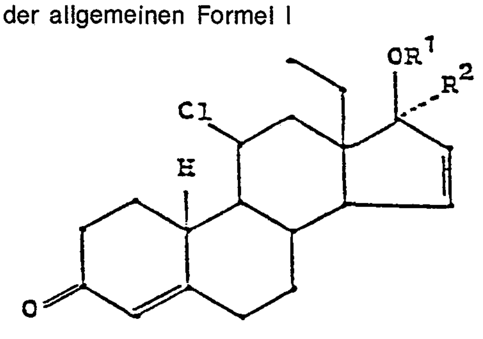

(I)

worin

$R^1$ ein Wasserstoffatom oder eine Acylgruppe einer organischen Carbonsäure mit bis zu 15 Kohlenstoffatomen und

$R^2$ eine Ethinyl-, Chlorethinyl- oder Propinylgruppe bedeuten.

2. 11β-Chlor-17α-ethinyl-17β-hydroxy-18-methyl-4,15-östradien-3-on.
3. 11β-Chlor-17α-chlorethinyl-17β-hydroxy-18-methyl-4,15-östradien-3-on.
4. 11β-Chlor-17β-hydroxy-18-methyl-17α-(1-propinyl)-4,15-östradien-3-on.
5. 17β-Acetoxy-11β-chlor-17α-ethinyl-18-methyl-4,15-östradien-3-on.
6. 17β-Butyryloxy-11β-chlor-17α-ethinyl-18-methyl-4,15-östradien-3-on.
7. 11β-Chlor-17α-ethinyl-17β-heptanoyloxy-18-methyl-4,15-östradien-3-on.
8. 11β-Chlor-17α-chlorethinyl-18-methyl-17β-propionyloxy-4,15-östradien-3-on.
9. 17β-Acetoxy-11β-chlor-18-methyl-17α-(1-propinyl)-4,15-östradien-3-on.
10. Pharmazeutische Präparate, gekennzeichnet durch einen Gehalt an Verbindungen gemäß Anspruch 1-9.
11. Verfahren zur Herstellung von 11β-Chlor-$\Delta^{15}$-Steroiden der allgemeinen Formel I

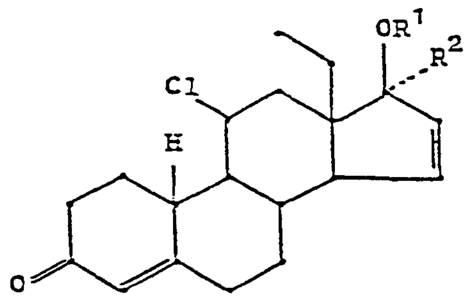

(I)

worin

$R^1$ ein Wasserstoffatom oder eine Acylgruppe einer organischen Carbonsäure mit bis zu 15 Kohlenstoffatomen und

$R^2$ eine Ethinyl-, Chlorethinyl- oder Propinylgruppe

bedeuten,

dadurch gekennzeichnet, daß man in das 17-Oxo-Steroid der Formel II

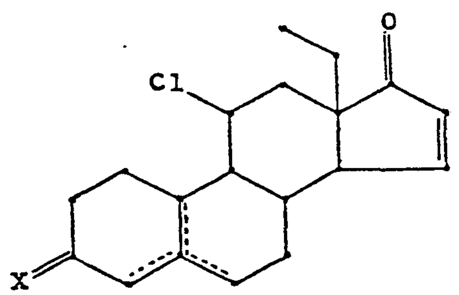

(II)

worin X eine freie Oxogruppe oder hydrolisierbare Oxoschutzgruppe darstellt und

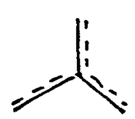

eine Doppelbindung in 4,5-, 5,6- oder 5,10-Stellung oder zwei von 3- und 5-Stellung ausgehende Doppelbindungen

bedeutet,

nach an sich bekannten Methoden mit Hilfe eines den Rest $R^2$ abgebenden Mittels diesen Rest unter Bildung eines tertiären Carbinols am 17-C-Atom einführt, die 3-Oxoschutzgruppe hydrolysiert und, je nach der letztlich gewünschten Bedeutung von $R^1$, gegebenenfalls die 17-Hydroxygruppe vor oder nach der Abspaltung der Oxoschutzgruppe verestert.

**Patentanspruch** (für den Vertragsstaat AT)

Verfahren zur Herstellung von 11β-Chlor-$\Delta^{15}$-Steroiden der allgemeinen Formel I

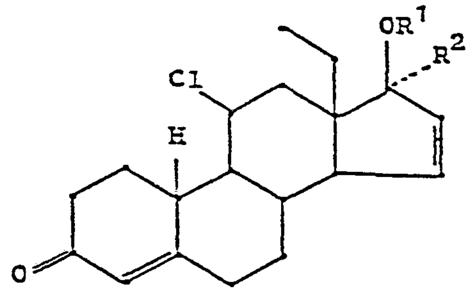

(I)

worin

$R^1$ ein Wasserstoffatom oder eine Acylgruppe einer organischen Carbonsäure mit bis zu 15 Kohlenstoffatomen und

$R^2$ eine Ethinyl-, Chlorethinyl- oder Propinylgruppe

bedeuten,

dadurch gekennzeichnet, daß man in das 17-Oxo-Steroid der Formel II

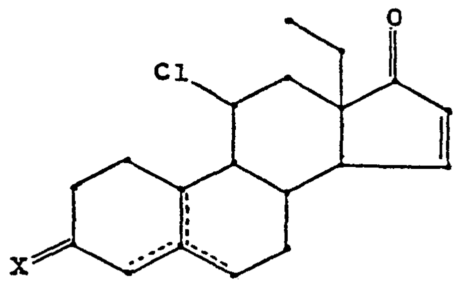

(II)

worin X eine freie Oxogruppe oder hydrolisierbare Oxoschutzgruppe darstellt und

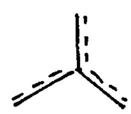

eine Doppelbindung in 4,5-, 5,6- oder 5,10-Stellung oder zwei von 3- und 5-Stellung ausgehende Doppelbindungen

bedeutet,

nach an sich bekannten Methoden mit Hilfe eines den Rest $R^2$ abgebenden Mittels diesen Rest unter Bildung eines tertiären Carbinols am 17-C-Atom einführt, die 3-Oxoschutzgruppe hydrolysiert und, je nach der letztlich gewünschten Bedeutung von $R^1$, gegebenenfalls die 17-Hydroxygruppe vor oder nach der Abspaltung der Oxoschutzgruppe verestert.

**Claims** (for the Contracting States : BE, CH, DE, FR, GB, IT, LI, LU, NL, SE)

1. 11β-Chloro-$\Delta^{15}$-steroids of general formula I

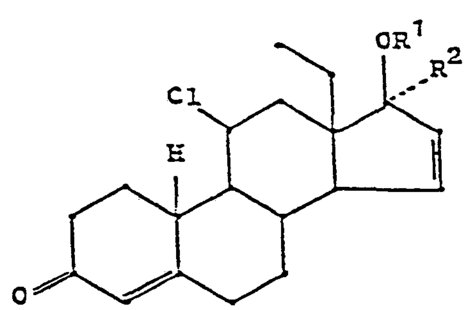

(I)

wherein

$R^1$ is hydrogen or an acyl group of an organic carboxylic acid of up to 15 carbon atoms and

$R^2$ is ethynyl, chloroethynyl or propynyl.

2. 11β-Chloro-17α-ethynyl-17β-hydroxy-18-methyl-4,15-oestradiene-3-one.

3. 11β-Chloro-17α-chloroethynyl-17β-hydroxy-18-methyl-4,15-oestradiene-3-one.

4. 11β-Chloro-17β-hydroxy-17α-chloroethynyl-18-methyl-17α-(1-propynyl)-4,15-oestradiene-3-one.

5. 17β-Acetoxy-11β-chloro-17α-ethynyl-18-methyl-4,15-oestradiene-3-one.

6. 17β-Butyryloxy-11β-chloro-17α-ethynyl-18-methyl-4,15-oestradiene-3-one.

7. 11β-Chloro-17α-ethynyl-17β-heptanoyloxy-18-methyl-4,15-oestradiene-3-one.

8. 11β-Chloro-17α-chloroethynyl-18-methyl-17β-propionyloxy-4,15-oestradiene-3-one.

9. 17β-Acetoxy-11β-chloro-18-methyl-17α-(1-propynyl)-4,15-oestradiene-3-one.

10. Pharmaceutical compositions characterised in that they comprise a compound according to claims 1 to 9.

11. Process for the preparation of 11β-chloro-$\Delta^{15}$-steroids of general formula I

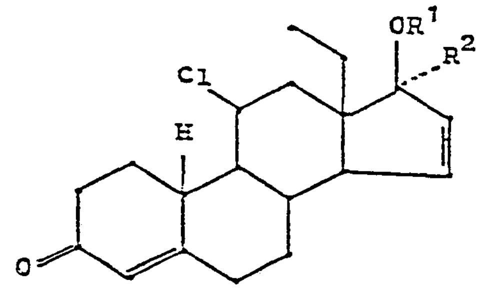

(I)

wherein

$R^1$ is hydrogen or an acyl group of an organic carboxylic acid of up to 15 carbon atoms and

$R^2$ is ethynyl, chloroethynyl or propynyl

characterised in that into a 17-oxosteroid of formula II

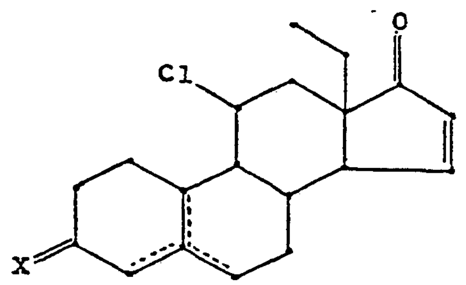

(II)

wherein, X is a free oxo group or a hydrolysable protected oxo group and

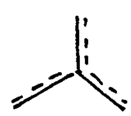

is a double bond in the 4,5-, 5,6- or 5,10-positions or two double bonds coming from the 3- and 5-positions.

there is introduced, in a known manner, on the $C_{17}$ atom, the group $R^2$, using an appropriate agent which delivers this group through formation of a tertiary carbinol, the 3-oxo protecting group is hydrolysed and depending on the finally desired meaning of $R^1$, the 17-hydroxy group is optionally esterified, before or after removal of the oxo protecting group.

**Claim** (for the Contracting State AT)

1. Process for the preparation of 11β-chloro-$\Delta^{15}$-steroids of general formula I

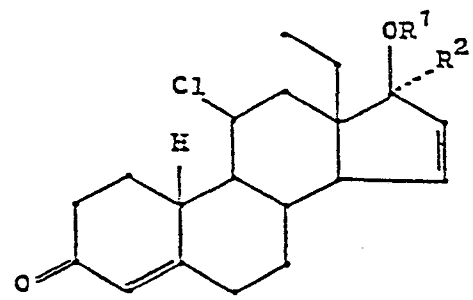

(I)

wherein

$R^1$ is hydrogen or an acyl group of an organic carboxylic acid of up to 15 carbon atoms and

$R^2$ is ethynyl, chloroethynyl or propynyl

characterised in that into a 17-oxosteroid of formula II

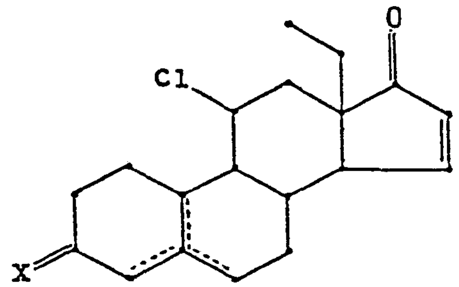

(II)

wherein, X is a free oxo group or a hydrolysable protected oxo group and

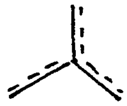

is a double bond in the 4,5-, 5,6- or 5,10-positions or two double bonds coming from the 3- and 5-positions.

there is introduced, in a known manner, on the $C_{17}$ atom, the group $R^2$, using an appropriate agent which delivers this group through formation of a tertiary carbinol, the 3-oxo protecting group is hydrolysed and depending on the finally desired meaning of $R^1$, the 17-hydroxy group is optionally esterified, before or after removal of the oxo protecting group.

**Revendications** (pour les Etats contractants : BE, CH, DE, FR, GB, IT, LI, LU, NL, SE)

1. 11β-Chloro-$\Delta^{15}$-stéroïdes répondant à la formule générale I

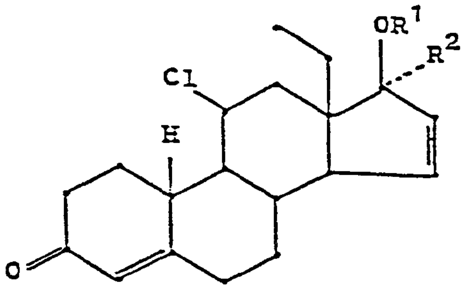

(I)

dans laquelle

$R^1$ représente un atome d'hydrogène ou un groupe acyle d'un acide carboxylique organique comportant jusqu'à 15 atomes de carbone

et $R^2$ représente un groupe éthynyle, chloroéthynyle ou propynyle.

2. La 11β-chloro-17α-éthynyl-17β-hydroxy-18-méthyl-4,15-œstradién-3-one.
3. La 11β-chloro-17α-chloroéthynyl-17β-hydroxy-18-méthyl-4,15-œstradién-3-one.
4. La 11β-chloro-17β-hydroxy-18-méthyl-17α-(1-propynyl)-4,15-œstradién-3-one.
5. La 17β-acétoxy-11β-chloro-17α-éthynyl-18-méthyl-4,15-œstradién-3-one.
6. La 17β-butyryloxy-11β-chloro-17α-éthynyl-18-méthyl-4,15-œstradién-3-one.
7. La 11β-chloro-17α-éthynyl-17β-heptanoyloxy-18-méthyl-4,15-œstradién-3-one.
8. La 11β-chloro-17α-chloroéthynyl-18-méthyl-17β-propionyloxy-4,15-œstradién-3-one.
9. La 17β-acétoxy-11β-chloro-18-méthyl-17α(1-propynyl)-4,15-œstradién-3-one.
10. Compositions pharmaceutiques, caractérisées par une teneur en les composés suivant l'une des revendications 1 à 9.
11. Procédé de préparation de 11β-chloro-$\Delta^{15}$-stéroïdes répondant à la formule générale I

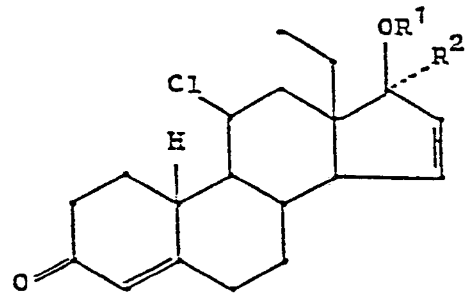

(I)

dans laquelle

$R^1$ représente un atome d'hydrogène ou un groupe acyle d'un acide carboxylique organique comportant jusqu'à 15 atomes de carbone,

et $R^2$ représente un groupe éthynyle, chloroéthynyle ou propynyle,

caractérisé en ce que, dans les 17-oxo-stéroïdes de la formule II

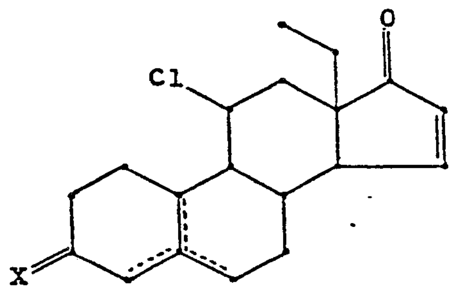

(II)

dans laquelle X représente un groupe oxo libre ou un groupe de protection d'oxo hydrolysable, et

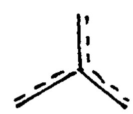

représente une double liaison en position 4,5, 5,6 ou 5,10 ou deux doubles liaisons partant de la position 3 et de la position 5,

on introduit suivant des procédés connus en soi, à l'aide d'un agent cédant le radical $R^2$, ce radical sur l'atome 17-C en formant un carbinol tertiaire, on hydrolyse le groupe de protection de 3-oxo et, selon la signification souhaitée en fin de réaction pour $R^1$, on estérifie éventuellement le groupe 17-hydroxy avant ou après la séparation du groupe de protection oxo.

**Revendication** (pour l'Etat contractant AT)

Procédé de préparation de 11β-chloro-$\Delta^{15}$-stéroïdes répondant à la formule générale I

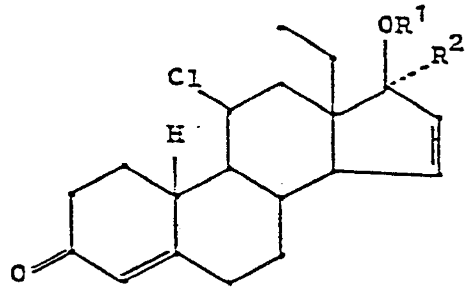

(I)

dans laquelle

$R^1$ représente un atome d'hydrogène ou un groupe acyle d'un acide carboxylique organique comportant jusqu'à 15 atomes de carbone,

et $R^2$ représente un groupe éthynyle, chloroéthynyle ou propynyle,

caractérisé en ce que, dans les 17-oxo-stéroïdes de la formule II

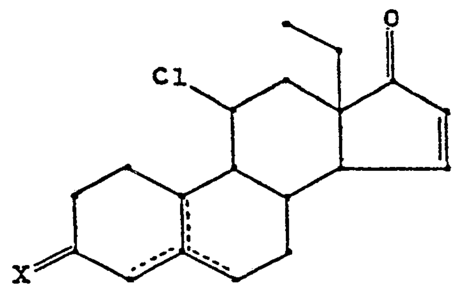

(II)

dans laquelle X représente un groupe oxo libre ou un groupe de protection d'oxo hydrolysable, et

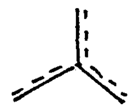

représente une double liaison en position 4,5, 5,6 ou 5,10 ou deux doubles liaisons partant de la position 3 et de la position 5,
on introduit suivant des procédés connus en soi, à l'aide d'un agent cédant le radical $R^2$, ce radical sur l'atome 17-C en formant un carbinol tertiaire, on hydrolyse le groupe de protection de 3-oxo et, selon la signification souhaitée en fin de réaction pour $R^1$, on estérifie éventuellement le groupe 17-hydroxy avant ou après la séparation du groupe de protection d'oxo.